(19) Europäisches Patentamt
European Patent Office
Office européen des brevets

(11) Veröffentlichungsnummer: **0 045 394 B1**

# (12) EUROPÄISCHE PATENTSCHRIFT

(45) Veröffentlichungstag der Patentschrift: **16.05.84**

(51) Int. Cl.$^3$: **C 07 D 231/12**

(21) Anmeldenummer: **81105338.8**

(22) Anmeldetag: **09.07.81**

(54) **Verfahren zur Herstellung von Pyrazol.**

(30) Priorität: **01.08.80 DE 3029160**

(43) Veröffentlichungstag der Anmeldung: **10.02.82 Patentblatt 82/6**

(45) Bekanntmachung des Hinweises auf die Patenterteilung: **16.05.84 Patentblatt 84/20**

(84) Benannte Vertragsstaaten: **AT BE CH DE FR GB IT LI NL SE**

(56) Entgegenhaltungen:
**DE - A - 2 441 504**
**DE - A - 2 602 965**

**JOURNAL OF GENERAL CHEMISTRY OF THE USSR Band 28, 1958, New York I. GRANDBERG et al. "Reactions of Hydrazine Derivatives. XX. Dehydrogenation of Pyrazolines" Seiten 3102 bis 3105 "Neuere Methoden der präparativen organischen Chemie" 2. Auflage, Teil 1 1944, VERLAG CHEMIE GMBH, Berlin**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Mangold, Dietrich, Dr., Hermann-Walker-Strasse 49, D-6903 Neckargemuend (DE)**
Erfinder: **Wahl, Josef, Bitzstrasse 25, D-6707 Schifferstadt (DE)**

**EP 0 045 394 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Pyrazol

Die Erfindung betrifft ein neues Verfahren zur Herstellung von Pyrazol.

Es sind bereits aus Chemistry of Carbon Compounds, Band IV a (Elsevier, N. Y., 1957), Seiten 246 bis 249, zahlreiche Synthesen von Pyrazolen bekannt, z. B. durch Umsetzung von Hydrazinen mit 1,3-Dicarbonylverbindungen, Dehydratation von Hydrazonen von $\beta$-Ketoestern, Cyclisierung von Hydrazonen von $\alpha$-Cyanoketonen, Umsetzung von Aldehydphenylhydrazonen mit $\beta$-Ketoestern in Gegenwart von Zinkchlorid, Kondensation von $\alpha$-Halogenhydrazonen mit Natrium-Ketonverbindungen oder von Diazoverbindungen mit Acetylenderivaten. Dje bedeutenderen Synthesewege führen über die Umsetzung von Derivaten von 1,3-Dicarbonylverbindungen mit Hydrazin oder Hydrazinderivaten in saurer, wäßriger Lösung oder mit Salzen des Hydrazins oder von Hydrazinderivaten zu verdünnten wäßrigen Lösungen von Pyrazolsalzen.

Bei all diesen Synthesen muß dabei, insbesondere im Fall des unsubstituierten Pyrazols auf schwierig zugängliche, aufwendig herzugstellende oder sicherheitstechnisch problematische Zwischenprodukte zurückgegriffen werden.

Probleme dieser Art treten in wesentlich geringerem Umfange auf, wenn Pyrazol durch Dehydrierung von Pyrazolin hergestellt werden kann.

Pyrazolin selbst ist aus technischen Einsatzstoffen leicht zugänglich, z. B. G. Wirsing — J. prakt. Chemie (2) 50, 538.

Bislang sind insbesondere für die Dehydrierung des unsubstituierten Pyrazolins nur Wege beschrieben worden, die vor allem unter dem Gesichtspunkt präparativer Durchführbarkeit nicht befriedigen konnten. Die Problematik wird von Grandberg und Kost (J. Gen. Chem. 28, 3102 (1958) ausführlich beschrieben. Diese Autoren entdeckten in der Umsetzung von elementarem Schwefel oder Selen mit Pyrazolinen eine Dehydriermethode, die bei zahlreichen Alkylpyrazolinen (Reaktionstemperatur 150 bis 250) in stark exothermer und offenbar auch im kleinen Labormaßstab schlecht kontrollierbarer Reaktion, Ausbeuten an Alkylpyrazolen von 65 bis 96% liefert.

Die beschriebene Methodik ist für die präparative Darstellung größerer Mengen (Maßstab der Beispiele 0,2 Mol) ungeeignet und keinesfalls auf technische Maßstäbe übertragbar, da die Ausbeuten des Verfahrens bei größeren Ansätzen stark zurückgehen.

Es wurde nun gefunden, daß sich Pyrazolin schon bei Temperaturen von 80—120°C in Pyrazol überführen läßt, wenn Schwefel bzw. Selen in Lösungsmitteln zur Dehydrierung eingesetzt wird, die Stickstoff im Molekül enthalten.

Das erhaltene Pyrazol ist sehr rein. Die Umsetzung läßt sich durch die folgende Reaktionsgleichung beschreiben:

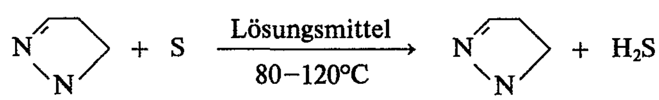


Die Menge des einzusetzenden Dehydrierungsmittels ist nicht kritisch, im allgemeinen wird mit Molverhältnissen von Pyrazolin zu Schwefel gleich 1 : 1 bis 1 : 3, vorzugsweise von 1 : 1 gearbeitet.

Auch die Menge des einzusetzenden Lösungsmittels oder des Lösungsmittelgemisches ist in weiten Grenzen variierbar; sie liegt vorzugsweise beim 0,5- bis 20fachen der eingesetzten Pyrazolinmenge.

Bei der Durchführung der Reaktion geht man zweckmäßigerweise so vor, daß man in die auf Reaktionstemperatur erwärmte Lösung oder Suspension des Schwefels oder Selens das Pyrazolin gleichmäßig einträgt, so daß der entweichende Schwefelwasserstoff (Selenwasserstoff) gut absorbiert bzw. aufgearbeitet werden kann. Die Reaktionsmischung wird bis zum Ende der Gasentwicklung weiter erwärmt und danach in üblicher Weise, z. B. durch Destillation oder Extraktion aufgearbeitet.

Als Lösungsmittel kommen beispielsweise in Frage: Aromatische Kohlenwasserstoffe, z. B. Toluol, Ehtylbenzol, o-, m-, p-Xylol, Isopropylbenzol, Methylnaphthalin; Halogenkohlenwasserstoffe, insbesondere Chlorkohlenwasserstoffe, z. B. Tetrachlorethylen, 1,1,2,2- oder 1,1,1,2-Tetrachlorethan, Amylchlorid, Dichlorpropan, Methylenchlorid, Dichlorbutan, Tetrachlorkohlenstoff, 1,1,1- oder 1,1,2-Trichlorethan, Trichlorethylen, Pentachlorethan, 1,2-Dichlorethan, 1,1-Dichlorethan, n-Propylchlorid, 1,2-cis-Dichlorethylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol; Ether, z. B. Ethylpropylether, Methyl-tert.-butylether, n-Butylethylether, Di-n-butylether, Diisobutylether, Diisoamylether, Diisopropylether, Cyclohexylmethylether, Diethylether, Ethylenglykoldimethylether, Tetrahydrofuran, Dioxan; Alkanole und Cycloalkanole wie Ethanol, Methanol, n-Butanol, Isobutanol, tert.-Butanol, Glykol, n-Propanol, Isopropanol, Amylalkohol, Cyclohexanol, 2-Methyl-4-pentanol, Ethylenglykolmonoethylether, 2-Ethylhexanol, Methylglykol, insbesondere solche mit 1 bis 4 Kohlenstoffatomen; aliphatische oder cycloaliphatische Kohlenwasserstoffe, z. B. Heptan, Pinan, Nonan, Benzinfraktionen innerhalb eines Siedepunktintervalls von 70 bis 190°C, Cyclohexan, Methylcyclohexan, Dekalin, Petrolether, Hexan Ligroin, 2,2,4-Trimethylpentan, 2,2,4-Trimethylpentan, 2,2,3-Trimethylpentan, 2,3,3-Trimethylpentan, Octan; und entsprechende Gemische.

Ferner können verwendet werden: stickstoffhaltige polare Lösungsmittel wie z. B. Pyridin und Alkylpyridin sowie technische Alkylpyridingemische, Alkylaminopyridine, Chinolin, und Alkylchinolin, Morpholin und Alkylmorpholin, alkylsubstituierte Harnstoffe wie Tetramethylharnstoff, N-Methylpyrrolidon, Amide wie Dimethylformamid oder Dibutylformamid, Ethylhexansäuredimethylamid, Tolysäurediethylamid, N,N-Dimethylimidazolidon Azole wie Pyrozol und Amine wie Tributyl- oder Tripropylamin. Auch schwefelhaltige Lösungsmittel wie Sulfolan oder Dimethylsulfoxid sind verwendbar.

Besonders bevorzugt sind Pyridin oder techn. Alkylpyridingemische und Mischungen von Lösungsmitteln, z. B. Kohlenwasserstoffen.

Bei Verwendung von Pyrazol als Lösungsmittel oder eines Lösungsmittelgemisches mit z. B. Toluol läßt sich das Verfahren besonders einfach durchführen.

Zweckmäßig verwendet man das Lösungsmittel in einer Menge von 400 bis 10 000 Gewichtsprozent, vorzugsweise von 100 bis 500 Gewichtsprozent, bezogen auf Pyrazolin.

Das neue Verfahren ist ohne Schwierigkeiten großtechnisch durchführbar und läßt sich sicher steuern.

Das nach dem Verfahren der Erfindung herstellbare Pyrazol ist ein wertvolles Ausgangsprodukt für die Herstellung von Pflanzenschutzmitteln, Pharmazeutika und Farbstoffen.

### Beispiel 1

In einer Rührapparatur werden 200 ml Pyridin und 16 g Schwefel (0,5 Mol) vorgelegt. Man erwärmt auf 110°C und tropft unter Rühren 35 g (0,5 Mol) 2-Pyrazolin gleichmäßig zu. Es erfolgt lebhafte Gasentwicklung. Nach Abklingen der Gasentwicklung wird unter vermindertem Druck destilliert. Man erhält bei Kp: 90 bis 100°C (4100 Pa) 31 g (92%) Pyrazol von ausgezeichneter Reinheit.

Analog erhält man mit jeweils 200 ml N-Methylmorpholin, n-Butanol bzw. Toluol 29 g (85%), 21,4 g (63%) bzw. 19,4 g (57%) farbloses Pyrazol.

### Beispiel 2

In einer Rührapparatur werden 1500 ml Toluol, 500 ml Pyridin und 160 g Schwefel vorgelegt und anschließend 350 g Pyrazolin bei 105 bis 110°C so in die Mischung eingetropft, daß eine gleichmäßiglebhafte Gasentwicklung stattfindet. Bis zum Abklingen der Gasentwicklung wird nachgerührt. Das Reaktionsgemisch wird im Vakuum destilliert. Man erhält 300 g (88%) fast farbloses Pyrazol, Fp = 68°C.

Ersetzt man das Pyridin durch 500 ml Pyrazol, so erhält man zusätzlich 283 g (83%) Pyrazol, Fp = 68°C.

### Beispiel 3

In einer Rührapparatur werden 180 ml Toluol, 20 g 4-Dimethylaminopyridin und 16 g Schwefel (0,5 Mol) vorgelegt. Man erwärmt auf 110°C und tropft unter Rühren 35 g (0,5 Mol) 2-Pyrazolin gleichmäßig zu. Es erfolgt lebhafte Gasentwicklung. Nach Abklingen der Gasentwicklung wird unter vermindertem Druck destilliert. Man erhält bei Kp 90—100°C (2700 Pa) 28 g (82%) Pyrazol.

## Patentansprüche

1. Verfahren zur Herstellung von Pyrazol durch Umsetzen von Pyrazolin mit Schwefel oder Selen, dadurch gekennzeichnet, daß man die Umsetzung bei 80—120°C in Lösungsmitteln durchführt, die Stickstoff im Molekül enthalten.

2. Verfahren gemäß Anspruch 1, dadurch gekennzeichnet, daß Pyridin, ggf. in Gemischen mit Kohlenwasserstoffen, als Lösungsmittel verwendet wird.

3. Verfahren gemäß Anspruch 1 bis 2, dadurch gekennzeichnet, daß Pyrazol, ggf. in Gemischen mit anderen Lösungsmitteln als Lösungsmittel verwendet wird.

## Claims

1. A process for the preparation of a pyrazole by reacting a pyrazoline with sulfur or selenium, wherein the reaction is carried out at from 80 to 120°C in a solvent which contains nitrogen in the molecule.

2. A process as claimed in claim 1, wherein a pyridine, or a mixture of a pyridine with a hydrocarbon, is used as the solvent.

3. A process as claimed in claim 1 or 2, wherein a pyrazole, or a mixture of a pyrazole with another solvent, is used as the solvent.

## Revendications

1. Procédé de préparation de pyrazol par réaction de pyrazoline avec du soufre ou du sélénium, caractérisé par le fait qu'on effectue la réction entre 80 et 120° C, dans des solvants qui centiennent de l'azote dans la molécule.

2. Procédé selon la revendication 1, caractérisé par le fait qu'on utilise, comme solvant, la pyridine, éventuelement en mélange avec des hydrocarbures.

3. Procédé selon la revendication 1 ou 2, caractérisé par le fait qu'on utilise, comme solvant, le pyrazol, éventuellement en mélange avec d'autres solvants.